# EUROPEAN PATENT APPLICATION

(11) **EP 4 578 470 A1**
(43) Date of publication of application: **02.07.2025**
(21) Application number: 23947274.9
(22) Date of filing: 07.10.2023
(51) Int. Cl.: A61L 27/36, A61L 27/50, A61L 33/10

(54) **NANOSTRUCTURED LOW-IMMUNOGENIC BIOLOGICAL ARTIFICIAL BLOOD VESSEL AND PREPARATION METHOD THEREFOR**

(30) Priority: 28.07.2023 CN 202310942899
(71) Applicant: Humatrix Medical Technology (Suzhou) Co., Ltd., Suzhou, Jiangsu 215000 (CN)
(72) Inventor: QIU, Xuefeng, Suzhou, Jiangsu 215000 (CN); GUO, Ying, Suzhou, Jiangsu 215000 (CN); WANG, Bo, Suzhou, Jiangsu 215000 (CN); ZHENG, Changdong, Suzhou, Jiangsu 215000 (CN); SUN, Jianling, Suzhou, Jiangsu 215000 (CN)
(74) Representative: HGF
(86) International application number: PCT/CN2023/123141
(87) International publication number: WO 2025/025335

(57) **Abstract**

A nanostructured low-immunogenic biological artificial blood vessel, a preparation method therefor and a use thereof. The preparation method comprises: decellularizing a pre-treated animal blood vessel to obtain a decellularized blood vessel; treating the decellularized blood vessel in an enzyme solution to obtain an enzyme-treated blood vessel, wherein the enzyme solution comprises nuclease and/or a biological enzyme; and using a cross-linking agent to cross-link the enzyme-treated blood vessel to obtain a nanostructured low-immunogenic biological artificial blood vessel, which is used as a blood vessel transplantation material. The artificial blood vessel overcomes the defects of decreased mechanical properties in decellularized biological tissues, in-vivo calcification after long-term use, and the presence of immunogenicity, antigenic components such as vascular wall cells and cell nuclei are fully removed, and original collagen and other extracellular matrix structural proteins in the tissue are retained to a greater extent, avoiding the in-vivo calcification of the blood vessel upon long-term implantation, enhancing the durability of use of the blood vessel; and the method has a short preparation cycle, low cost and high long-term patency rate.

## Description

This application claims the priority of Chinese Patent Application No. 202310942899.8, filed with the China National Intellectual Property Administration on July 28, 2023, and titled with "NANOSTRUCTURED LOW-IMMUNOGENIC BIOLOGICAL ARTIFICIAL BLOOD VESSEL AND PREPARATION METHOD THEREFOR", the disclosure of which is hereby incorporated by reference in its entirety.

### FIELD

The present disclosure relates to the technical field of medical devices, in particular to a biological artificial blood vessel having a nanostructure and low immunogenicity and a production method thereof.

### BACKGROUND

Nowadays, according to the statistics of the World Health Organization, the prevalence of cardiovascular diseases is increasing with the development of society, which have become one of the killers endangering human health and the leading cause of death among humans. According to the statistics, there are currently more than 11 million coronary heart disease cases in China, with more than 5 million patients requiring revascularization. The number of coronary artery bypass grafting surgeries in China reached nearly 100,000 in 2022, a ten-fold increase from 10 years ago. In 2019, there were approximately 45.3 million patients with peripheral atherosclerosis in China, with about 8% of patients requiring revascularization. However, the current penetration rate of surgery, including the interventional surgery, is only 0.3%, indicating a huge market space. China National Renal Data System (CNRDS) shows that the hemodialysis rate is increasing year by year (increasing by 13% to 14%/year). In China, there are currently over 130 million patients with chronic renal insufficiency, with more than 3 million patients requiring hemodialysis. The number of registered patients undergoing dialysis was about 200,000 in 2011, 630,000 in 2019, 690,000 in 2020 and 750,000 in 2021, and it is estimated that the number will be about 1.27 million in 2027 and more than 1.6 million in 2030. Among them, more than 80% of the patients received arteriovenous fistula creation by conventional surgery, 12% received central venous catheters, and about 7.2% received artificial vascular access (CNRDS data published at the 6th Summit Forum on Nephrology, Renal Intervention, and Blood Purification Devices held in Pengcheng in November 2022). In the United States, there are about 650,000 patients undergoing long-term chronic haemodialysis per year. Among them, about 65% of the patients received arteriovenous fistula creation by conventional surgery, 15% received central venous catheters, and about 20% received artificial vascular access. After the creation of an arteriovenous fistula by conventional surgery, it is necessary for patients to wait 3 to 6 months for the fistula to mature before it can be used as a dialysis access. In China, one-third to one-half of patients may experience failed fistula maturation 3-6 months after the surgical creation of the arteriovenous fistula, and thus cannot receive dialysis. Therefore, only small-caliber artificial blood vessels can be used, but this access is prone to aneurysms formation and then deterioration. Central venous catheters can only be used as a temporary dialysis access, which shows an annual infection rate of 200%, many complications and a high mortality in patients. Traditional expanded polytetrafluoroethylene (ePTFE) artificial blood vessels are susceptible to infection and formation of intraluminal thrombosis, with a 1-year patency rate of about 10-30% and a service life of about 2 years, severely limiting clinical outcomes. Therefore, the development of small-caliber vessels suitable for coronary artery disease, lower limb artery replacement, and haemodialysis vascular access in patients with end-stage renal disease is of great clinical significance.

Vascular repair and replacement is one of the most effective options for the treatment of vascular diseases. Currently, blood vessel sources generally include autologous blood vessels, artificial blood vessels made of polymer materials and foreign blood vessels, and tissue-engineered blood vessels are in the research and development stage. Autologous internal mammary artery or radial artery shows an excellent medium and long-term patency rate, and the coronary artery bypass grafting thereof has a 10-year patency rate of about 90% or more. Autologous great saphenous vein shows a 5-year patency rate of about 60-70% and a 10-year patency rate of about 40-50%, and their calibers are only matched for coronary artery bypass grafting and not for other clinical indications, for which reason they cannot be used in other clinical indications. In addition, the source of autologous blood vessels is limited, and 10-30% of patients cannot provide great saphenous veins due to varicose great saphenous veins or obesity, diabetes and other factors. The harvest of saphenous vein may cause secondary trauma and complications such as incision infection, lower limb edema, and prolongation of surgery and anaesthesia, which affects the postoperative recovery of patients. Although the artificial blood vessel made of polymer material is easy to be produced commercially on a large scale, and the caliber and length thereof can be easily controlled, it has a low long-term patency rate, is prone to infection and deterioration, and has a short service life, which seriously affects the clinical effect.

Foreign blood vessels are classified into allogeneic and xenogeneic blood vessels. Allogeneic blood vessels are of restricted origin and access for ethical and regulatory reasons. Xenogeneic blood vessels are generally of animal origin, taken directly from mammals, and can be used as vascular grafts for lower limb arterial replacement, coronary artery bypass grafting, and dialysis.

In addition to blood vessels, another medical device of animal origin, the bioprosthetic heart valve (BHV), does not require lifelong anticoagulation after implantation in the human body. However, calcification of the valve leaflets after implantation of the BHV into the body can lead to structural deterioration of the valve, causing valvular stenosis and/or regurgitation, and the deleterious immune response of the human body to xenoantigens in the BHV is involved in the occurrence of calcification and structural deterioration, limiting its durability in vivo and affecting longevity.

Recently, a research article entitled with "The role of antibody responses against glycans in bioprosthetic heart valve calcification and deterioration" has been published on Nature Medicine, which investigated the mechanisms of haemodynamics, immune response and leaflet deterioration in 1,668 patients up to 15 years after BHV implantation. The results of the study showed that the BHV implantation caused an increase in anti-α-Gαl and anti-Neu5Gc during the first six months after implantation, and that the control group did not have significantly elevated levels of either antibody (lower than those of patients implanted with BHV). The α-Gαl is found in a large number of non-primate mammals, and anti-α-Gal antibodies are present in humans. The combination of α-Gal antigens with anti-α-Gal antibodies forms an immune barrier against xenograft. In order to ensure the safe and effective use of medical devices of animal origin, materials of animal origin should be selected, and appropriate processes should be used to reduce or remove α-Gal antigen.

Mdical devices of animal origin such as bioprosthetic heart valve and biological blood vessels currently on the market have been chemically treated to substantially remove immunogenicity, but some cellular antigens still remain. Therefore, medical devices of animal origin need to be completely decellularized and cross-linked to improve mechanical strength before use. However, existing decellularization methods may destroy the extracellular matrix components of the animal-derived vascular wall during the treatment process and lead to incomplete removal of cells and antigens from the wall, which can easily lead to calcification and deterioration in clinical use and affect the in vivo reconstruction of blood vessels and the long-term patency rate.

The use of conventional cross-linking agents is also another very important factor in inducing calcification. Current experiments have shown the following facts: 1) Calcification occurred after the implantation of BHV into the body mainly involves passive mechanisms, wherein xenogenic cells, residual cellular components, and cell membrane-associated phospholipid molecules react with calcium ions, serving as a major factor in inducing calcification. Cross-linking agents react with the amino groups in the collagen of valve material through their aldehyde groups to undergo a Schiff base reaction, forming a five-membered ring of carbon atoms, thereby stabilizing collagen, resisting tissue enzymolysis, and enhancing mechanical properties. However, it can cause cell death in the biological valve tissue, leaving residual cells, and disrupt the calcium pumps in cell membranes, increasing the chance of calcium ions interacting with phospholipids. 2) Residual cross-linking agent in the product can combine with calcium ions directly. 3) Elastin and glycosaminoglycan cannot be fixed, which reduces valve durability and promotes calcification. Metalloproteinase and cathepsin can promote the degradation of elastin and the generation of proinflammatory peptides, and induces calcium deposition. 4) The number of amino acid residues decreases after the treatment cross-linking agents, the phosphoric acid bonds of collagen are exposed, and the negative charge on the valve surface is increased, which binds to positively charged calcium ions. 5) BHV does not have the anti-calcification barrier provided by endothelial cells presented on the surface of human valves. As a result, the adhesion of platelet and fibrin, along with the deposition of calcium and phosphate, leads to calcification. Research has demonstrated that removing the elastin component from the tissue can significantly reduce the occurrence of calcification after implantation of valve into the body.

It has been clinically demonstrated that vascular calcification is harmful to patients with chronic kidney disease. In patients with chronic renal failure, the vascular calcification rate is very high, leading to poor patient prognosis. A Chinese dialysis calcification study, according to the four-year follow-up result, showed that the incidence of vascular calcification increased from 77.4% in the first year to 90% in the fourth year.

Therefore, the development of a biological blood vessel suitable for haemodialysis patients is of important clinical value in improving the quality of life of patients, reducing the number of interventions and prolonging the lives of patients.

The present disclosure provides a biological artificial blood vessel having a nanostructure and low immunogenicity and a production method thereof. Wherein a blood vessel is decellularized using a decellularizing agent that has little impact on the extracellular matrix of the blood vessel wall to preserve as much as possible the extracellular matrix of the blood vessel and the vessel wall structure without affecting the mechanical properties and compliance of the blood vessel. Then the residual cell nucleus and the antigenic components after the decellularization are removed by enzyme treatment. The use of a novel cross-linking agent increases the mechanical properties of the decellularized blood vessels. The resulting blood vessel has excellent biocompatibility, very low incidence of vascular calcification and improved long-term patency rate.

### SUMMARY

**In** view of this, the technical problem to be solved by the present disclosure is to provide a biological artificial blood vessel having a nanostructure and low immunogenicity and a production method thereof. The biological artificial blood vessel produced by this method has good mechanical properties, a low immunogenicity, a low incidence of vascular calcification and a significantly improved long-term patency rate.

The present disclosure provides a method for producing a biological artificial blood vessel having a nanostructure and low immunogenicity, comprising:
S1) decellularizing a pretreated animal blood vessel to obtain a decellularized blood vessel,
S2) treating the decellularized blood vessel with an enzyme solution to obtain an enzyme-treated blood vessel, wherein the enzyme solution comprises a nuclease and/or a biological enzyme, and
S3) crosslinking the enzyme-treated blood vessel with a cross-linking agent to obtain the biological artificial blood vessel having a nanostructure and low immunogenicity.

Preferably, the animal blood vessel is an artery or a vein of a large animal, wherein the large animal is selected from the group consisting of a pig, a sheep, a dog, a cow and a horse.

The animal blood vessel is selected from the group consisting of an aorta, a pulmonary artery, a superior artery, an inferior artery, a common carotid artery, an internal jugular vein, an external jugular vein, a femoral artery, a femoral vein, an iliac artery, an iliac vein, a superior mesenteric artery, an inferior mesenteric artery, a rectal artery, a median sacral artery and a lower limb artery.

The decellularization in step S1 is carried out using a decellularizing agent, the decellularizing agent comprises a detergent or comprises a combination of a detergent and a chelating agent, wherein the detergent in the decellularizing agent has a concentration of 0.01 to 500 mmol/L, and the chelating agent in the decellularizing agent has a concentration of 0.01 to 500 mmol/L.

The decellularization is carried out at a temperature of 10°C to 38°C for 2 to 72 h, and the decellularizing agent is changed every 1 to 24 h during the decellularization.

Preferably, in the case that the decellularizing agent comprises a detergent, the detergent is one or more selected from the group consisting of a non-ionic detergent, an anionic detergent and a cationic detergent, and an amphoteric detergent.

In the case that the decellularizing agent comprises a combination of a detergent and a chelating agent, the detergent is one or more selected from the group consisting of a non-ionic detergent, an anionic detergent, a cationic detergent and an amphoteric detergent.

Wherein, the non-ionic detergent is one or more selected from the group consisting of polyethylene glycol, polyethylene glycol octylphenyl ether, polyol, polyoxyethylene fatty alcohol ether and polyoxyethylene alkyl phenol ether.

The anionic detergent is one or more selected from the group consisting of sodium dodecyl sulfate, lithium dodecyl sulfate, sodium dodecyl sulfonate, sodium cholate and sodium deoxycholate.

The cationic detergent is selected from the group consisting of benzalkonium bromide, cetyltrimethylammonium bromide and a combination thereof.

The amphoteric detergent is selected from the group consisting of 3-[(3-cholamidopropyl)-dimethylammonium]-1-propanesulfonate inner salt, n-tetradecyl-N,N-dimethyl-3-ammonium-1-propanesulfonate and a combination thereof.

The chelating agent is selected from the group consisting of an inorganic chelating agent, an organic chelating agent and a combination thereof.

Wherein, the inorganic chelating agent is one or more selected from the group consisting of sodium tripolyphosphate, sodium hexametaphosphate and sodium pyrophosphate.

The organic chelating agent is one or more selected from the group consisting of amino triacetic acid, ethylenediaminetetraacetic acid, ethylene glycol bis(tetraacetic acid), ethylenediamine diacetic acid, cyclohexane diaminetetraacetic acid, S,S-ethylenediamine disuccinic acid, diethyl triacetic acid, diethylenetriamine pentaacetic acid and a salt thereof, citric acid, tartaric acid, gluconic acid, hydroxyethyl ethylenediamine triacetic acid and dihydroxyethyl glycine.

The decellularizing agent further comprises sodium chloride, sodium hydroxide and water.

Wherein, the sodium chloride in the decellularizing agent has a concentration of 0.01 to 1.0 mmol/L.

The sodium hydroxide in the decellularizing agent has a concentration of 0.1 to 2.0 mol/L.

Preferably, the nuclease in the enzyme solution has a concentration of 1 to 50,000 KU/L, and/or the biological enzyme in the enzyme solution has a concentration of 1 to 50,000 KU/L.

The enzyme solution further comprises 1 to 50 wt% human serum or animal serum.

The enzyme solution further comprises physiological saline or buffer.

The enzyme treatment in step S2) is carried out at a temperature of 36°C to 37°C for 2 to 72 h, and the enzyme solution is changed every 1 to 24 h during the enzyme treatment.

Preferably, after the enzyme treatment, step S2) further comprises treating the blood vessel with a solution comprising a biological enzyme to obtain an enzyme-treated blood vessel.

The nuclease in the enzyme treatment is selected from the group consisting of DNase, RNase and a combination thereof, the biological enzyme in both the enzyme treatment and the solution comprising a biological enzyme is one or more selected from the group consisting of pepsin, lipase, trypsin, cathepsin, papain, ficain and subtilisin, the biological enzyme in the solution comprising a biological enzyme has a concentration of 1 to 50,000 KU/L, and the solution comprising a biological enzyme further comprises a solvent selected from the group consisting of physiological saline, buffer and a special solution for enzyme preparation.

The treatment with the solution comprising a biological enzyme is carried out at a temperature of 36°C to 37°C for 0.25 to 48 h, and the solution comprising a biological enzyme is changed every 1 to 24 h during the treatment.

Preferably, the cross-linking agent is one or more selected from the group consisting of a glutaraldehyde solution, an oxidized starch solution, a dialdehyde starch solution and a Jeffamine buffer.

The glutaraldehyde solution has a concentration of 0.1 to 30 wt%.

The oxidized starch solution has a concentration of 0.01 to 10 wt%.

The dialdehyde starch solution has a concentration of 0.01 to 10 wt%.

The Jeffamine buffer has a concentration of 0.01 to 1.0 mol/L.

Preferably, after the crosslinking, step S3) further comprises performing covalent binding of heparin to the cross-linked blood vessel by a chemical method to obtain a biological artificial blood vessel.

Preferably, the covalent binding of heparin to the cross-linked blood vessel by a chemical method is carried out using a solution of carbodiimide, N-hydroxysulfosuccinimide and heparin in MES buffer, and the heparin in the solution for the covalent binding of heparin has a concentration of 1 to 200 mg/ml.

The present disclosure further provides a biological artificial blood vessel having a nanostructure and low immunogenicity produced by the method above, comprising a vascular collagen or a nanofibrous structure formed by vascular collagen and elastin.

Preferably, the vascular collagen includes type I collagen, type III collagen and/or type IV collagen.

In the case that the biological artificial blood vessel comprises only the vascular collagen, the vascular collagen has a content of 30% to 85% of the dry weight of the biological artificial blood vessel.

In the case that the biological artificial blood vessel comprises the vascular collagen and elastin, the vascular collagen has a content of 20% to 55% of the dry weight of the biological artificial blood vessel, and the elastin has a content of 20% to 55% of the dry weight of the biological artificial blood vessel.

The present disclosure further provides use of the biological artificial blood vessel having a nanostructure and low immunogenicity above in the manufacture of a vascular graft material for hemodialysis vascular access in chronic renal failure, a vascular graft material for arterial trauma of lower limbs, a vascular graft material for peripheral artery bypass grafting or a vascular graft material for coronary artery bypass grafting.

The present disclosure further provides use of the biological artificial blood vessel having a nanostructure and low immunogenicity above in the manufacture of a vascular graft material for vascular access of patients with chronic haemodialysis; wherein, the biological artificial blood vessel is used as a replacement repair graft material for failure, infection or aneurysm formation after chronic dialysis autologous arteriovenous fistula creation or artificial vascular fistula creation.

The present disclosure provides a method for producing a biological artificial blood vessel having a nanostructure and low immunogenicity, comprising: S1) decellularizing a pretreated animal blood vessel to obtain a decellularized blood vessel, S2) treating the decellularized blood vessel with an enzyme solution to obtain an enzyme-treated blood vessel, wherein the enzyme solution comprises a nuclease and/or a biological enzyme, and S3) crosslinking the enzyme-treated blood vessel with a cross-linking agent to obtain the biological artificial blood vessel having a nanostructure and low immunogenicity. Compared with the prior art, the present disclosure adopts a decellularization treatment that has little impact on the extracellular matrix of the blood vessel wall to remove cells while preserving as much as possible the extracellular matrix of the blood vessel and the vessel wall structure without affecting the mechanical properties and compliance of the blood vessel. Then the residual cell nucleus and antigenic components after the decellularization are removed by enzyme treatment, which significantly reduces the immunogenicity of the decellularized blood vessel. Finally, the decellularized blood vessels are treated by a cross-linking agent to increase the mechanical properties of the decellularized blood vessels and block antigenic sites of xenoprotein, which further reduces the immunogenicity, increases the durability of the blood vessel after implantation into the body, and reduces vascular thrombosis formation and the incidence of vascular calcification and deterioration. The method for producing a biological artificial blood vessel provided by the present disclosure completely removes cells while reducing the damage to extracellular matrix of blood vessel wall and effect on mechanical properties, which is conducive to the adhesion and growth of endothelial cells after implantation of the biological artificial blood vessel into the body and improves the long-term patency rate. This method has a short production cycle and low cost, and can be used to produce clinically used biological artificial blood vessels with various calibers and lengths.

In addition, the biological artificial blood vessel having a nanostructure and low immunogenicity produced by the method provided by the present disclosure solves deficiencies of reduced mechanical properties of biological tissue due to decellularization, calcification in vivo after long-term use and immunogenicity. The method fully removes vessel wall cells, cell nucleus and other antigenic components while preserving original extracellular matrix structural proteins in tissues to a great extent, such as collagen. In the method, the antigens of xenoproteins are blocked by a cross-linking agent to avoid blood vessel calcification after long-term implantation in the body and increase the durability of the blood vessel. Moreover, this method has a short production cycle, a low cost and a high long-term patency rate, which is suitable for commercial production.

### BRIEF DESCRIPTION OF DRAWINGS

FIG. 1A and FIG. 1B show histological images of the biological blood vessel treated with 5.2% ficin for 1/2 h in Example 1 of the present disclosure.
FIG. 2A, FIG. 2B, FIG. 2C and FIG. 2D show histological images of the biological blood vessel treated with 5.2% ficin for 3 h in Example 1 of the present disclosure.
FIG. 3A shows histological images of the biological blood vessel decellularized by the decellularization method A in Example 1 of the present disclosure.
FIG. 3B shows histological images of the biological blood vessel decellularized by the decellularization method B in Example 1 of the present disclosure.
FIG. 4A shows the results of collagen staining of the biological blood vessel decellularized by the decellularization method A in Example 1 of the present disclosure.
FIG. 4B shows the results of elastin staining of the biological blood vessel decellularized by the decellularization method A in Example 1 of the present disclosure.
FIG. 5A shows the results of collagen staining of the biological blood vessel decellularized by the decellularization method B in Example 1 of the present disclosure.
FIG. 5B shows the results of elastin staining of the biological blood vessel decellularized by the decellularization method B in Example 1 of the present disclosure.
FIG. 6 is a photograph of an untreated fresh blood vessel in Example 1 of the present disclosure.
FIG. 7 is a photograph of the biological artificial blood vessel obtained in Example 1 of the present disclosure.
FIG. 8A, FIG. 8B, FIG. 8C and FIG. 8D show scanning electron microscope (SEM) cross-sectional images at different magnifications of the biological artificial blood vessel obtained in Example 1 of the present disclosure.
FIG. 9A, FIG. 9B, FIG. 9C and FIG. 9D show SEM images at different magnifications of the tunica intima of the biological artificial blood vessel obtained in Example 1 of the present disclosure.
FIG. 10A, FIG. 10B, FIG. 10C and FIG. 10D show SEM images at different magnifications of the tunica adventitia of the biological artificial blood vessel obtained in Example 1 of the present disclosure.
FIG. 11 shows the result of DAPI staining of the untreated fresh blood vessel in Example 1 of the present disclosure.
FIG. 12 shows the result of DAPI staining of the biological artificial blood vessel obtained in Example 1 of the present disclosure.
FIG. 13 shows the measurement results of the radial tensile strength of the biological artificial blood vessels in Examples 3, 4, 5 and 6 of the present disclosure.
FIG. 14 shows the measurement results of the burst pressure of the biological artificial blood vessels in Examples 3, 4, 5 and 6 of the present disclosure.
FIG. 15 shows the image of the e-PTFE control during the animal experimental operation in Example 7 of the present disclosure.
FIG. 16 shows the image of the biological artificial blood vessel during the animal experimental operation in Example 7 of the present disclosure.
FIG. 17 shows an ultrasound image of the biological artificial blood vessel after the animal experimental operation in Example 7 of the present disclosure.
FIG. 18 shows an ultrasound image of the blood vessel at the arterial anastomosis after the animal experimental operation in Example 7 of the present disclosure.
FIG. 19 shows the patency of the vascular graft as examined by arteriovenous fistula angiography after the animal experimental operation in Example 7 of the present disclosure.
FIG. 20 shows a picture of the right common carotid artery replacement during the animal experimental operation in Example 7 of the present disclosure.
FIG. 21 shows an angiogram of the right common carotid artery replacement after the animal experimental operation in Example 7 of the present disclosure.
FIG. 22 is a follow-up angiogram of the right common carotid artery replacement after the animal experimental operation in Example 7 of the present disclosure, showing the patency of the biological artificial blood vessel and the autologous blood vessel near the anastomosis.

### DETAILED DESCRIPTION

The embodiments of the present disclosure will be clearly and completely described in conjunction with the examples of the present disclosure below. It is apparent that the described examples are only some of the embodiments, rather than all of the embodiments of the present disclosure. All other embodiments obtained by those skilled in the art based on the embodiments of the present disclosure without making any inventive effort fall within the protection scope of the present disclosure.

The present disclosure provides a method for producing a biological artificial blood vessel having a nanostructure and low immunogenicity, comprising: S1) decellularizing a pretreated animal blood vessel to obtain a decellularized blood vessel, S2) treating the decellularized blood vessel with an enzyme solution to obtain an enzyme-treated blood vessel, wherein the enzyme solution comprises a nuclease and/or a biological enzyme, and S3) crosslinking the enzyme-treated blood vessel with a cross-linking agent to obtain the biological artificial blood vessel having a nanostructure and low immunogenicity.

Wherein, the source of the raw materials used for the animal blood vessels of the present disclosure is in accordance with the China national standards of medical devices for animal origin on animal breeding.

In the present disclosure, the animal blood vessel is preferably an artery or a vein of a large animal selected from the group consisting of a pig, a sheep, a dog, a cow and a horse; and the animal blood vessel includes, but is not limited to, an aorta, a pulmonary artery, a superior artery, an inferior artery, a common carotid artery, an internal jugular vein, an external jugular vein, a femoral artery, a femoral vein, an iliac artery, an iliac vein, a superior mesenteric artery, an inferior mesenteric artery, a superior rectal artery, an inferior rectal artery, a median sacral artery and a lower limb artery. The pretreated animal blood vessel is obtained by pretreating an animal blood vessel by a method known to those skilled in the art, comprising sterilizing the obtained animal blood vessel and removing fat and connective tissue around the blood vessel without damaging the tunica externa and the blood vessel wall.

The pretreated animal blood vessel is decellularized to obtain a decellularized blood vessel. The decellularization is carried out using a decellularizing agent, and the decellularizing agent preferably comprises a detergent or a combination of a detergent and a chelating agent. In the case that the decellularizing agent comprises a detergent, the detergent is preferably one or more selected from the group consisting of a non-ionic detergent, an anionic detergent and a cationic detergent, and an amphoteric detergent. The molar ratio of the one or more selected from the group consisting of a non-ionic detergent, an anionic detergent and a cationic detergent to the amphoteric detergent is preferably (1-3):(5-15), more preferably (1.5-2.5):(6-12), still more preferably (1.5-2.5):(6-10) and most preferably (1.8-2.5):(8-10). In the case that the decellularizing agent comprises a combination of a detergent and a chelating agent, the detergent is preferably one or more selected from the group consisting of a non-ionic detergent, an anionic detergent, a cationic detergent and an amphoteric detergent. Wherein, the non-ionic detergent is preferably one or more selected from the group consisting of polyethylene glycol, polyethylene glycol octylphenyl ether, polyol, polyoxyethylene fatty alcohol ether and polyoxyethylene alkyl phenol ether, the anionic detergent is preferably one or more selected from the group consisting of sodium dodecyl sulfate, lithium dodecyl sulfate, sodium dodecyl sulfonate, sodium cholate and sodium deoxycholate, the cationic detergent is preferably selected from the group consisting of benzalkonium bromide, cetyltrimethylammonium bromide and a combination thereof, and the amphoteric detergent is preferably selected from the group consisting of 3-[(3-cholamidopropyl)-dimethylammonium]-1-propanesulfonate inner salt, n-tetradecyl-N,N-dimethyl-3-ammonium-1-propanesulfonate and a combination thereof. The detergent in the decellularizing agent has a concentration of preferably 0.01 to 500 mmol/L, more preferably 1 to 100 mmol/L, still more preferably 1 to 50 mmol/L, and most preferably 1 to 20 mmol/L. In an embodiment provided by the present disclosure, the detergent in the decellularizing agent has a specific concentration of 9.8 mmol/L, 8 mmol/L, 10 mmol/L or 6 mmol/L. The chelating agent is preferably selected from the group consisting of an inorganic chelating agent, an organic chelating agent and a combination thereof. The inorganic chelating agent is preferably one or more selected from the group consisting of sodium tripolyphosphate, sodium hexametaphosphate and sodium pyrophosphate, and the organic chelating agent is preferably one or more selected from the group consisting of amino triacetic acid, ethylene diaminete traacetic acid, ethylene glycol bis(tetraacetic acid), ethylene diamine diacetic acid, cyclohexane diamine tetraacetic acid, S,S-ethylenediamine disuccinic acid, diethyl triacetic acid, diethylenetriamine pentaacetic acid and a salt thereof, citric acid, tartaric acid, gluconic acid, hydroxyethyl ethylenediamine triacetic acid and dihydroxyethyl glycine. The chelating agent in the decellularizing agent has a concentration of preferably 0.01 to 500 mmol/L, more preferably 1 to 300 mmol/L, still more preferably 1 to 100 mmol/L, still more preferably 1 to 50 mmol/L, and most preferably 10 to 30 mmol/L. In an embodiment provided by the present disclosure, the chelating agent in the decellularizing agent has a specific concentration of 25 mmol/L, 30 mmol/L or 20 mmol/L. In the present disclosure, the decellularizing agent further comprises sodium chloride, sodium hydroxide and water. The sodium chloride in the decellularizing agent has a concentration of preferably 0.01 to 1.0 mmol/L, more preferably 0.01 to 0.5 mmol/L, still more preferably 0.08 to 0.2 mmol/L, still more preferably 0.1 to 0.15 mmol/L, and most preferably 0.1 to 0.12 mmol/L. In an embodiment provided by the present disclosure, the sodium chloride in the decellularizing agent has a specific concentration of 0.12 mmol/L, 0.15 mmol/L or 0.1 mmol/L. The sodium hydroxide in the decellularizing agent has a concentration of preferably 0.1 to 2 mol/L, more preferably 0.8 to 1.5 mol/L, still more preferably 0.8 to 1.3 mol/L, and most preferably 1 to 1.3 mol/L. In an embodiment provided by the present disclosure, the sodium hydroxide in the decellularizing agent has a specific concentration of 1 mol/L, 1.2 mol/L or 0.8 mol/L. The decellularization is carried out at a temperature of preferably 10°C to 38°C, more preferably 20°C to 38°C, still more preferably 25°C to 38°C, and most preferably 36°C to 38°C, and the decellularization is preferably carried out for 2 to 72 h, more preferably for 8 to 48 h, still more preferably for 10 to 45 h, still more preferably for 15 to 40 h, still more preferably for 20 to 40 h, still more preferably for 25 to 35 h, and most preferably for 30 h. During the decellularization, the decellularizing agent is changed preferably every 1 to 24 h, more preferably every 2 to 12 h, still more preferably every 2 to 8 h, and most preferably every 5 to 8 h. In an embodiment provided by the present disclosure, the decellularizing agent is changed every 2 to 8 h. The decellularizing agent is changed preferably 1 to 6 times, more preferably 2 to 6 times, and still more preferably 3 to 6 times.

In the present disclosure, after the decellularization is completed, preferably, the treated blood vessel is washed in physiological saline or buffer to obtain a decellularized blood vessel, wherein the physiological saline is preferably 0.9% (V/V) saline, and the buffer is preferably PBS buffer. The washing is carried out at a temperature of preferably 10°C to 38°C, more preferably 20°C to 38°C, still more preferably 25°C to 38°C, and most preferably 36°C to 38°C. The washing is preferably carried out for 10 to 60 min, more preferably for 20 to 50 min, still more preferably for 35 to 45 min, and most preferably for 40 min.

The decellularized blood vessel is enzymatically digested with an enzyme solution, and the enzyme solution comprises a nuclease and/or a biological enzyme. The nuclease is preferably selected from the group consisting of DNase, RNase and a combination thereof. In an embodiment provided by the present disclosure, the nuclease is specifically DNase II, DNase I, or Benzonase nuclease. The biological enzyme is preferably one or more selected from the group consisting of pepsin, lipase, trypsin, cathepsin, papain, ficain and subtilisin. The nuclease in the enzyme solution has a concentration of preferably 1 to 50,000 KU/L, more preferably 1 to 4000 KU/L, still more preferably 5 to 1000 KU/L, and most preferably 5 to 100 KU/L, and/or, the biological enzyme in the enzyme solution has a concentration of preferably 1 to 50,000 KU/L, more preferably 100 to 50,000 KU/L, and still more preferably 500 to 10000 KU/L. In an embodiment provided by the present disclosure, the nuclease and/or biological enzyme in the enzyme solution has a specific concentration of 3 KU/L, 9 KU/L, 15 KU/L, 20 KU/L, 50 KU/L, 5000 KU/L, 1000 KU/L, or 20,000 KU/L. The enzyme solution preferably further comprises 1 to 10 wt% serum, physiological saline or buffer. The serum is preferably human serum or animal serum, and the serum has a concentration of preferably 1 to 30 wt%, more preferably 4 to 20 wt%, still more preferably 4 to 10 wt%, still more preferably 4 to 6 wt%, and most preferably 5 wt%. The physiological saline is preferably 0.9% (V/V) saline, and the buffer is preferably PBS buffer. The enzyme treatment is carried out at a temperature of preferably 36°C to 37°C. The enzyme treatment is preferably carried out for 2 to 72 h, more preferably for 12 to 72 h, still more preferably for 24 to 72 h, still more preferably for 36 to 60 h, still more preferably for 40 to 50 h, and most preferably for 48 h. During the enzyme treatment, the enzyme solution is changed preferably every 1 to 24 h, more preferably every 5 to 10 h, and still more preferably every 6 to 8 h. The enzyme solution is changed preferably 1 to 8 times, more preferably 3 to 7 times, still more preferably 4 to 6 times, and most preferably 5 times. In the case that the enzyme treatment is carried out with an enzyme solution comprising nuclease, the method preferably comprises a treatment with a solution comprising a biological enzyme after the enzyme treatment to obtain an enzyme-treated blood vessel. The biological enzyme in the solution comprising a biological enzyme is one or more selected from the group consisting of pepsin, lipase, trypsin, cathepsin, papain, ficain and subtilisin. The biological enzyme in the solution comprising a biological enzyme has a concentration of preferably 1 to 50,000 KU/L, more preferably 1 to 10,000 KU/L, still more preferably 1 to 5000 KU/L, and most preferably 100 to 1000 KU/L. Alternatively, the biological enzyme in the solution comprising a biological enzyme has a concentration in mass percent of preferably 1 to 10 wt%, more preferably 3 to 8 wt%, still more preferably 5 to 6 wt%, and most preferably 5.2 to 5.5 wt%. In an embodiment provided by the present disclosure, the biological enzyme in the solution comprising a biological enzyme has a specific concentration of 3 KU/L, 1000 KU/L or 100 KU/L. The solution comprising a biological enzyme further comprises a solvent selected from the group consisting of physiological saline, buffer and a special solution for enzyme preparation. The physiological saline is preferably 0.9% (V/V) physiological saline, and the buffer is preferably PBS buffer. The treatment with the solution comprising a biological enzyme is carried out at a temperature of preferably 36°C to 37°C, and the treatment is preferably carried out for 0.25 to 48 h, more preferably for 6 to 48 h, still more preferably for 12 to 48 h, still more preferably for 20 to 36 h, and most preferably for 24 to 36 h. During the treatment, the solution comprising a biological enzyme is changed preferably every 1 to 24 h, more preferably every 5 to 15 h, still more preferably every 5 to 10 h, and most preferably every 6 to 8 h. During the treatment, the solution comprising a biological enzyme is changed preferably 1 to 5 times, and more preferably 2 to 3 times.

In the present disclosure, after the enzyme treatment or after the treatment with the solution comprising a biological enzyme, the method preferably comprises washing with physiological saline or buffer to obtain an enzyme-treated blood vessel. The physiological saline is preferably 0.9% (V/V) physiological saline, and the buffer is preferably a PBS buffer. The washing is carried out at a temperature of preferably 10°C to 38°C, more preferably 20°C to 38°C, still more preferably 25°C to 38°C, and most preferably 36°C to 38°C. The washing is preferably carried out for 10 to 60 min, more preferably for 20 to 50 min, still more preferably 35 to 45 min, and most preferably for 40 min.

The enzyme-treated blood vessel is cross-linked with a cross-linking agent. The cross-linking agent is preferably one or more selected from the group consisting of a glutaraldehyde solution, an oxidized starch solution, a dialdehyde starch solution and a Jeffamine buffer. The glutaraldehyde solution has a concentration of preferably 0.1 to 30 wt%, more preferably 1 to 10 wt%, still more preferably 3 to 8 wt%, still more preferably 4 to 6 wt%, and most preferably 5 wt%. The glutaraldehyde solution has a pH value of preferably 7 to 11, more preferably 8 to 10, and still more preferably 9. The oxidized starch solution has a concentration of preferably 0.01 to 10 wt%, more preferably 1 to 5 wt%, still more preferably 2 to 4 wt%, and most preferably 3 wt%. The oxidized starch solution has a pH value of preferably 7 to 11, more preferably 8 to 10, and still more preferably 9. The dialdehyde starch solution has a concentration of preferably 0.01 to 15 wt%, more preferably 1 to 10 wt%, still more preferably 2 to 8 wt%, and most preferably 6 wt%. The dialdehyde starch solution has a pH value of preferably 7 to 11, more preferably 8 to 10, and still more preferably 9. The Jeffamine buffer has a concentration of preferably 0.01 to 1.0 mmol/L, more preferably 0.03 to 0.08 mmol/L, still more preferably 0.04 to 0.06 mmol/L, and most preferably 0.05 mmol/L. The Jeffamine buffer has a pH value of preferably 8 to 13, more preferably 9 to 12, and still more preferably 10 to 11. The cross-linking treatment is carried out at a temperature of preferably 20°C to 30°C, more preferably 23°C to 27°C, and still more preferably 25°C. The cross-linking treatment is preferably carried out for 12 to 36 h, more preferably for 18 to 30 h, and still more preferably for 20 to 24 h.

The cross-linked blood vessel is preferably covalently bound with heparin by a chemical method, to modify the surface of the vascular lumen. The covalent binding of heparin to the cross-linked blood vessel by a chemical method is carried out using a solution of carbodiimide, N-hydroxysulfosuccinimide and heparin in MES buffer. The carbodiimide in the solution has a concentration of preferably 1 to 100 mg/ml, more preferably 10 to 80 mg/ml, still more preferably 20 to 70 mg/ml, and most preferably 30 to 50 mg/ml. The N-hydroxysulfosuccinimide in the solution has a concentration of preferably 1 to 100 mg/ml, more preferably 1 to 80 mg/ml, still more preferably 5 to 60 mg/ml, and most preferably 10 to 50 mg/ml. The heparin in the solution has a concentration of preferably 1 to 200 mg/ml, more preferably 10 to 150 mg/ml, still more preferably 20 to 100 mg/ml, and most preferably 30 to 80 mg/ml. The heparin in the solution for covalent binding of heparin to the cross-linked blood vessel by a chemical method has a pH value of preferably 5 to 13, more preferably 6 to 12, still more preferably 7 to 11. The covalent binding of heparin is carried out at a temperature of preferably 20°C to 37°C, more preferably 23°C to 30°C, and still more preferably 25°C. The covalent binding of heparin is carried out for preferably 0.5 to 24 h, more preferably for 0.5 to 12 h, and still more preferably for 0.5 h to 5 h.

The blood vessel covalently bound with heparin is preferably washed with physiological saline or buffer to obtain the biological artificial blood vessel having a nanostructure and low immunogenicity, which has anticoagulant activity by heparin on the inner wall of the vascular lumen. The physiological saline is preferably 0.9% (V/V) physiological saline, and the buffer is preferably PBS buffer. The washing is carried out at a temperature of preferably 10°C to 38°C, more preferably 20°C to 38°C, still more preferably 25°C to 38°C, and most preferably 36°C to 38°C. The washing is preferably carried out for 10 to 60 min, more preferably for 20 to 50 min, still more preferably for 35 to 45 min, and most preferably for 40 min.

In the present disclosure, unless otherwise specified, all the reagents mentioned above are sterilized by filtration.

The present disclosure adopts a decellularization treatment method with little impact on the extracellular matrix of the vessel wall. The method removes cells while preserving as much as possible the extracellular matrix of the blood vessel and the structure of the vessel wall without affecting the mechanical properties and compliance of the blood vessel. Then the residual cell nucleus and antigenic components after the decellularization are removed by enzyme treatment, which significantly reduces the immunogenicity of the decellularized blood vessel. Finally, the decellularized blood vessels are treated by a cross-linking agent to increase the mechanical properties of the decellularized blood vessels and block antigenic sites of xenoprotein, which further reduces the immunogenicity, increases the durability of the blood vessel after implantation into the body, and reduces vascular thrombosis formation and the incidence of vascular calcification and deterioration. The method for producing a biological artificial blood vessel provided by the present disclosure completely removes cells while reducing the damage to extracellular matrix of blood vessel wall and effect on mechanical properties, which is conducive to the adhesion and growth of endothelial cells after implantation of the biological artificial blood vessel into the body and improves the long-term patency rate. This method has a short production cycle and low cost, and can be used to produce clinically used biological artificial blood vessels with various calibers and lengths, which are suitable for commercial production.

The present disclosure further provides a biological artificial blood vessel having a nanostructure and low immunogenicity produced by the method described above. The biological artificial blood vessel comprises a vascular collagen or a nanofibrous structure formed by vascular collagen and elastin, and further comprises a small amount of fibronectin, laminin, proteoglycans, and glycoproteins. The mechanical properties of the biological artificial blood vessel are better than those of the human autologous great saphenous vein and similar or superior to those of human small-caliber arteries.

The biological artificial blood vessel provided by the present disclosure can be implanted in the body for a long time.

According to the present disclosure, specifically, the vascular collagen includes type I collagen, type **III** collagen and/or type IV collagen.

In the case that the biological artificial blood vessel of the present disclosure mainly comprises only the vascular collagen, the vascular collagen has a content of 30% to 85% of the dry weight of the biological artificial blood vessel.

In the case that the biological artificial blood vessel of the present disclosure mainly comprises the vascular collagen and elastin, the vascular collagen has a content of 20% to 55% of the dry weight of the biological artificial blood vessel, and the elastin has a content of 20% to 55% of the dry weight of the biological artificial blood vessel.

The present disclosure further provides use of the biological artificial blood vessel having a nanostructure and low immunogenicity in a clinical indication including the establishment of hemodialysis vascular access in chronic renal failure, vascular replacement for arterial trauma of lower limbs, peripheral artery bypass grafting, coronary artery bypass grafting and caliber-matched vascular replacement or bypass grafting in other parts of the human body.

The present disclosure further provides use of the biological artificial blood vessel having a nanostructure and low immunogenicity in the establishment of vascular access of patients with chronic haemodialysis; wherein, the biological artificial blood vessel is used as a replacement repair graft material for failure, infection or aneurysm formation after chronic dialysis autologous arteriovenous fistula creation or artificial vascular fistula creation. The product can be used for haemodialysis rapidly after implanted into the body, as early as 1-10 days after operation, avoiding the 3-4 week waiting period of traditional polymer blood vessels.

The biological artificial blood vessel provided by the present disclosure can be rapidly endothelialized after implanted into the body to reduce vascular thrombosis formation, and its near- to mid-term and long-term patency rate in vivo is higher than that of traditional polymer materials such as expanded polytetrafluoroethylene (ePTFE), and other small-caliber blood vessels such as polyurethane. In addition, the biological artificial blood vessel of the present disclosure has very low immunogenicity, is resistant to infection, allows rapid healing with the surrounding tissues of the organism, and reduces or avoids leakage of plasma or blood from the vessel wall.

In order to further illustrate the present disclosure, the method for producing a biological artificial blood vessel having a nanostructure and low immunogenicity provided by the present disclosure is described in detail below in conjunction with examples.

The reagents used in the following examples are all commercially available.

In the present disclosure, the directly harvested blood vessel is decellularized, treated with enzyme and cross-linked to obtain the xenogeneic decellularized matrix vessel. That is, the present disclosure provides a biological artificial blood vessel having a nanostructure and low immunogenicity and a production method thereof. The method specifically comprises the following steps:

(1) sterilizing a blood vessel harvested directly from a slaughterhouse, removing the fat and connective tissue surrounding the blood vessel with a medical scissor to obtain a pre-treated blood vessel,

(2) decellularizing the blood vessel with a chemical reagent to remove cellular components and obtain an xenogeneic decellularized matrix vessel,

(3) treating the decellularized matrix vessel with a nuclease to remove residual cell nucleus and antigenic components, alternatively, further treating the decellularized matrix vessel with a biological enzyme to remove elastin on the vessel wall, and

(4) treating the enzyme-treated decellularized matrix vessel with a cross-linking agent solution containing aldehydes, oxidized starch and/or dialdehyde starch to improve the mechanical properties of the blood vessel and block xenogeneic protein antigenic sites on extracellular matrix while maintaining vascular compliance, to obtain the final biological artificial blood vessel having a nanostructure and low immunogenicity.

### Example 1

(1) Bovine arteries were harvested from an 800 kg healthy cattle with inspection passed in a slaughterhouse. The inner caliber of the blood vessels was 3 to 10 mm. The fat and connective tissue surrounding the blood vessels were carefully removed using medical scissors without damaging the outer membrane of the blood vessels to obtain the pretreated blood vessels.

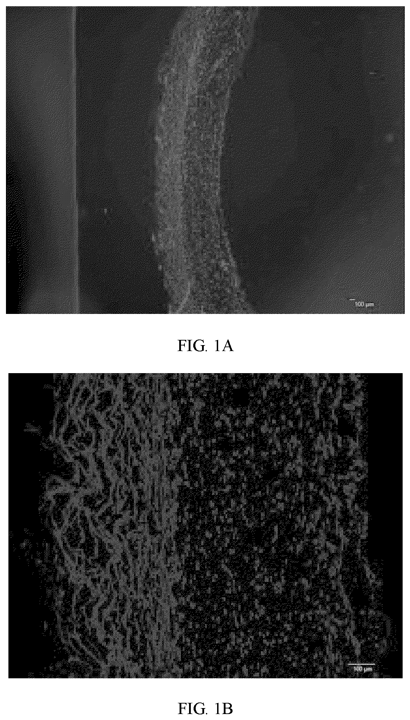

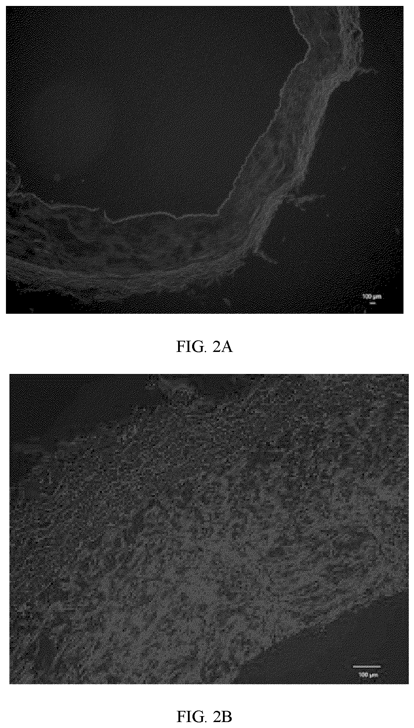

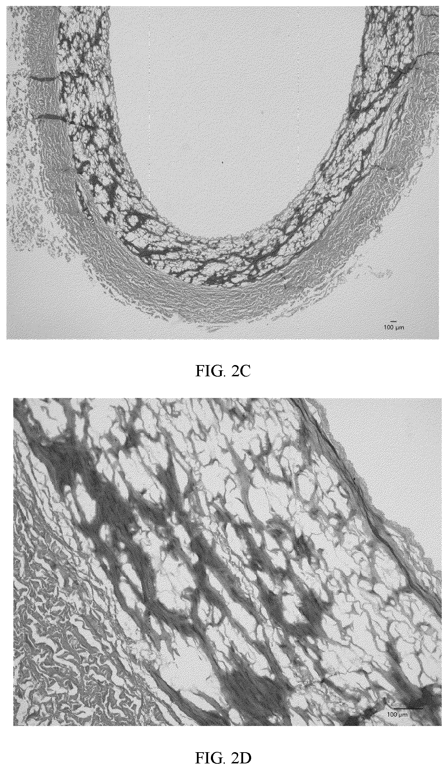

The pretreated blood vessels were treated with 5.2% ficin (Sigma) at 37°C, and samples were taken for testing at each time point of 1/2 h, 3 h, 9 h, 12 h and 24 h. The solution was changed every 8 h after 8 h. The blood vessels were then transferred to 0.9% (V/V) physiological saline and washed at 37°C for 40 min to obtain the enzyme-treated blood vessels, which were then subjected to tissue sectioning, staining, DNA quantification, and quantification of elastin of the vessel walls. The results show that a large number of cells (FIG. 1a and FIG. 1b) and elastin (Table 1) were still present in the vessel walls after 1/2 h of the enzyme treatment, substantially no elastin or trace amount of elastin was present in the vessel walls after 3 h of the enzyme treatment (Table 1), while numerous nuclei (FIG. 2a and FIG. 2b) and residual DNA (FIG. 2c and FIG. 2d) were still present in the vessel walls, leading to a strong immune response in the clinical application. Decellularization was therefore necessary prior to the enzyme treatment.

**Table 1 Comparison of quantification of elastin and collagen levels, and radial mechanical property of bovine arteries treated with ficin at different time points**

| Time (h) | 1/2 | 3 | 9 | 12 | 24 |
|---|---|---|---|---|---|
| DNA quantification results (ng/mg) | 1687.13±35.46 | 1741.20±109.11 | 1550.30±109.47 | 1712.86±174.14 | 2329.90±104.25 |
| Elastin (percentage by dry weight)/% | 21.31±3.56 | 0.30±0.06 | 0.15±0.01 | 0.06±0.01 | 0 |
| Collagen (percentage by dry weight)/% | 45.50±7.64 | 71.01±5.18 | 72.60±8.2 | 74.10±3.07 | 76.86±7.04 |
| Radial tensile strength (N/mm) | 2.72±0.16 | 2.51±0.3 | 1.99±0.34 | 1.62±0.09 | 1.36±0.21 |

### (2) Two decellularization processes

A. The pretreated blood vessels were placed in a decellularizing agent of 3-[(3-cholamidopropyl)-dimethylammonium]-1-propanesulfonate inner salt (8 mmol/L), sodium dodecyl sulfate (1.8 mmol/L), NaCl (0.12 mmol/L) and NaOH (1 mol/L) in sterile deionized water, and were treated at 37°C for 12 h. The solution was changed every 2 h for a total of 6 changes. After the treatment was completed, the blood vessels were transferred to 0.9% (V/V) physiological saline and washed at 37°C for 40 min to obtain the decellularized blood vessels.

B. The pretreated blood vessels were placed in a decellularizing agent of 3-[(3-cholamidopropyl)-dimethylammonium]-1-propanesulfonate inner salt (8 mmol/L), EDTA (8 mmol/L), NaCl (0.12 mmol/L) and NaOH (1 mol/L) in sterile deionized water, and were treated at 37°C for 12 h. The solution was changed every 2 h for a total of 6 changes. After the treatment was completed, the blood vessels were transferred to 0.9% (V/V) physiological saline and washed at 37°C for 40 min to obtain the decellularized blood vessels.

The blood vessels treated by two different decellularization processes were separately subjected to radial tensile and burst pressure measurement according to the method of "YY/T 0500-2021 Cardiovascular Implants and Extracorporeal Systems-Vascular Prostheses-Tubular Vascular Grafts and Vascular Patches". The vascular tissues decellularized by the process A showed a radial tensile strength of 2.92±0.42 N/mm (n=8) and a burst pressure of 2642.4±116.7 mmHg; and the vascular tissues decellularized by the process B showed a radial tensile strength of 2.55±2.50 N/mm (n=8) and a burst pressure of 2014.2±121 mmHg, indicating that the decellularization process A can provide a better vascular mechanical property compared to the process B.

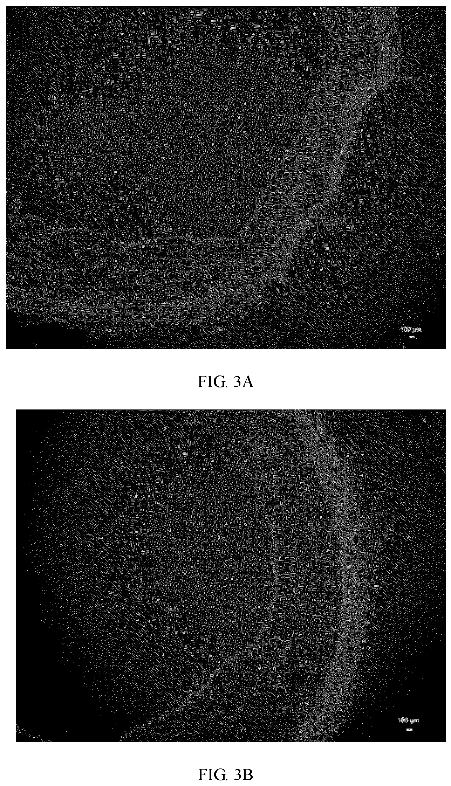

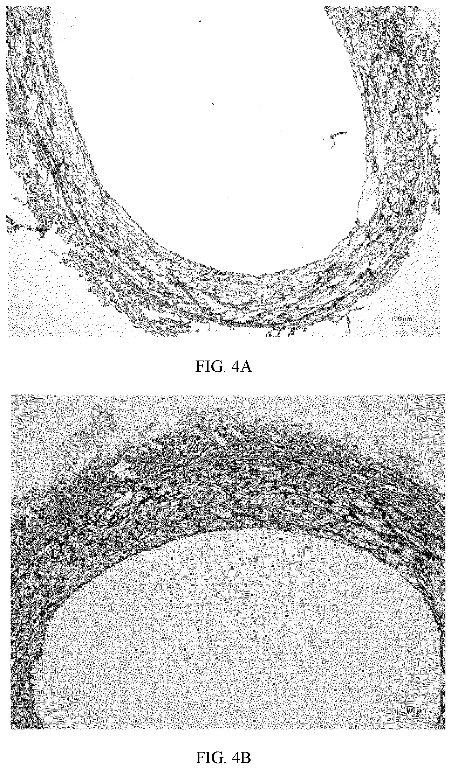

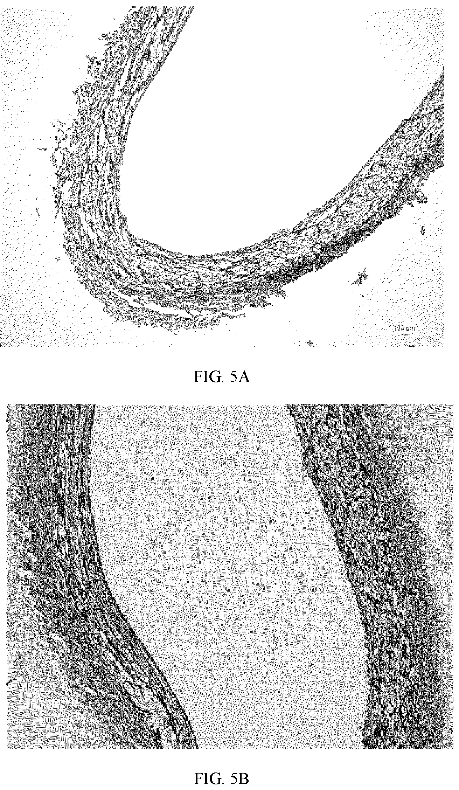

(3) Enzyme Treatment: The decellularized blood vessels of the previous step were placed in 0.9% (V/V) physiological saline containing DNase I (9 KU/L), and treated at 37°C for 48 h. The solution was changed every 8 h for a total of 5 changes. After the treatment was completed, the blood vessels were transferred to 0.9% (V/V) physiological saline and washed for 40 min at 37°C to obtain DNase I-treated blood vessels, which were then subjected to histological analysis and DNA quantitation assay. The results showed no residual nuclei and DNA was present in the vessel walls (FIG. 3a and FIG. 3b show the results of enzyme-treated blood vessels decellularized by the processes A and B, respectively), and the vessel walls mainly contained collagen and elastin. FIG. 4 and FIG. 5 show the results of histological staining of the biological blood vessels decellularized by the decellularization processes A and B, respectively. FIG. 4a shows the results of collagen staining of the biological blood vessels decellularized by the decellularization process A. FIG. 4b shows the results of elastin staining of the biological blood vessels decellularized by the decellularization process A. FIG. 5a shows the results of collagen staining of the biological blood vessels decellularized by the decellularization process B. FIG. 5b shows the results of elastin staining of the biological blood vessels decellularized by the decellularization process B. Decellularization process A can preserve the mechanical properties of the vessel wall tissue and completely remove vessel wall cells and DNA after the treatment of DNase I. Process A was preferably used for later experiments.

The blood vessels decellularized by process A above were treated with DNase I and then with 5.2% ficin (Sigma) in 0.9% (V/V) saline at 37°C, and samples were taken for testing at each time point of 1/2 h, 3 h, 9 h, 12 h and 24 h. The solution was changed every 8 h after 8 h. After the above treatment was completed, the blood vessels were then transferred to 0.9% (V/V) physiological saline and washed at 37°C for 40 min to obtain the enzyme-treated vascular grafts.

The vascular tissues treated with ficin for different durations were subjected to quantification of DNA, fibrin and elastin and radial tensile property test, and the results are shown in Table 2. Subsequently, 3 h of ficin treatment was preferably used for later experiments.

**Table 2 Results of quantification of elastin and fibrin and radial tensile property test of vascular tissues treated with ficin for different durations**

| Time (h) | 1/2 | 3 | 9 | 12 | 24 |
|---|---|---|---|---|---|
| DNA quantification results (ng/mg) | 62.43±4.83 | 66.84±29.41 | 68.41±8.44 | 33.45±17.38 | 59.06±41.83 |
| Elastin (percentage by dry weight)/% | 49.33±6.09 | 72.85±5.09 | 74.95±4.02 | 77.30±7.83 | 79.25±6.17 |
| Collagen (percentage by dry weight)/% | 19.61±2.86 | 0.22±0.03 | 0.07±0.01 | 0.01±0.00 | 0 |
| Radial tensile strength (N/mm) | 2.86±0.47 | 3.39±0.28 | 3.38±0.43 | 3.41±0.37 | 2.78±0.27 |

(4) Cross-linking Treatment: Finally, the blood vessels treated with DNase I for 48 h and ficin for 3 h in step (3) were placed in a solution containing 6% (wt) dialdehyde starch, treated at 25°C for 30 h, then transferred to 0.9% (V/V) physiological saline and washed for 40 min to obtain biological artificial blood vessels.

(5) Covalent Binding of Heparin: 1200 mg of EDC and 1000 mg of Sulfo-NHS were dissolved in 20 ml of MES buffer, 1000 mg of heparin was dissolved in another 20 ml of MES buffer, the two solutions were mixed in an equal volume, and the resulting mixture was adjusted to a pH of 7 to obtain a heparin grafting agent. The biological blood vessels obtained in the previous step were placed in the heparin grafting agent and shaken in the dark for 3 h to obtain the biological blood vessels coated with heparin, which were then implanted into the body to perform surgical procedures of venous fistula creation and vascular replacement.

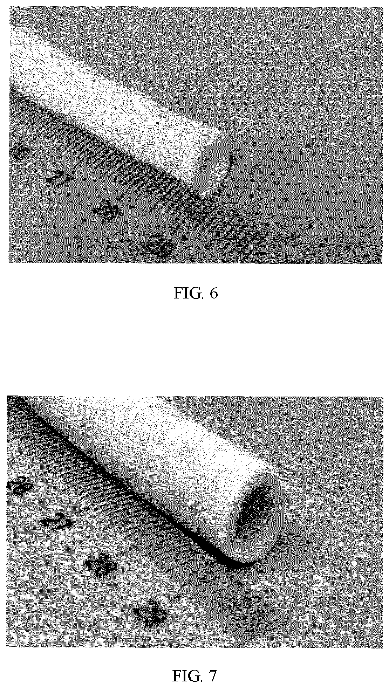

(6) The radial tensile strengths and burst pressures of untreated fresh blood vessels (FIG. 6) and treated biological artificial blood vessels in step (5) (FIG. 7) were measured and compared. The results showed that the untreated fresh blood vessels had a radial tensile strength of 2.15±0.22 N/mm² (n=8), and the treated biological artificial blood vessels had a radial tensile strength of 2.05±0.13 N/mm² (n=8), showing no significant difference (P>0.05). The untreated fresh blood vessels had a burst pressure of 2252.5±220.8 mmHg (n=8), and the treated biological artificial blood vessels in step (5) had a burst pressure of 2388.8±433.3 mmHg (n=8), showing no significant difference (P>0.05). The results above indicated that the decellularization did not affect the mechanical properties of blood vessels.

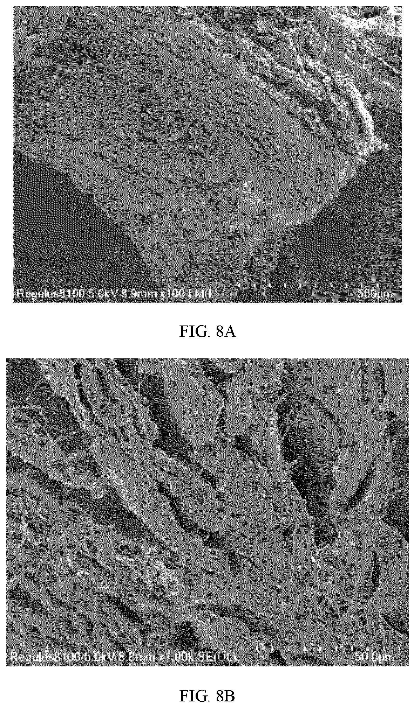

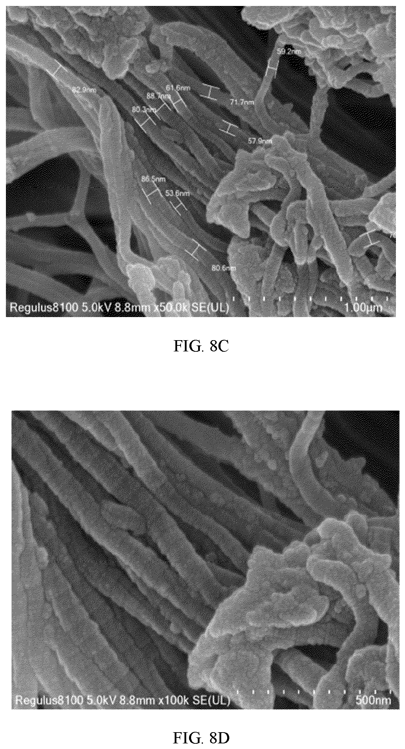

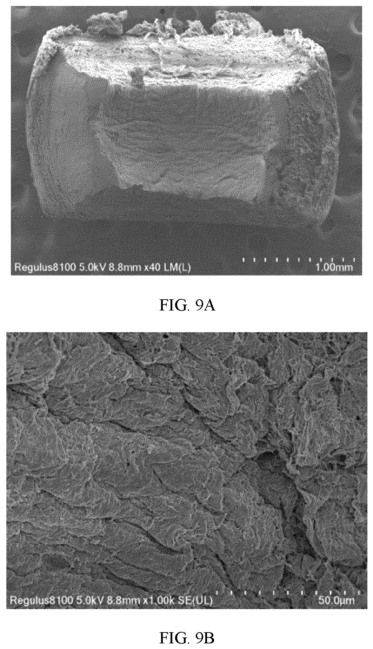

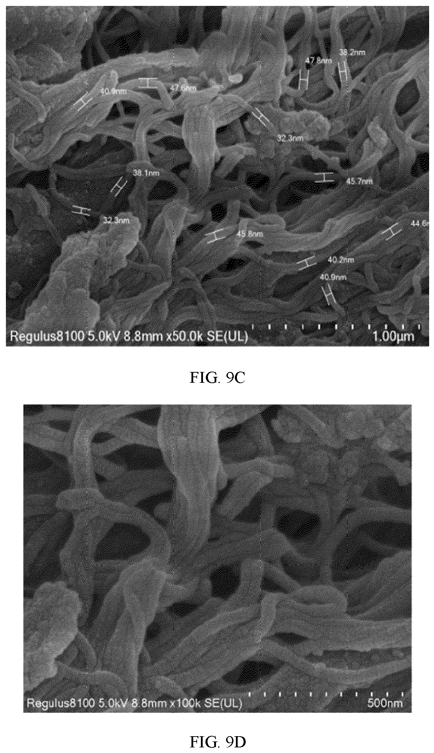

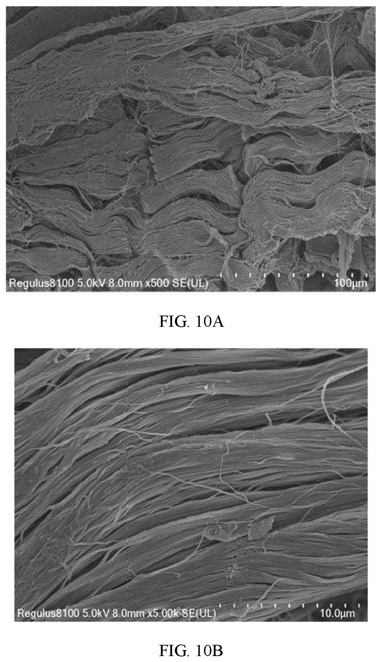

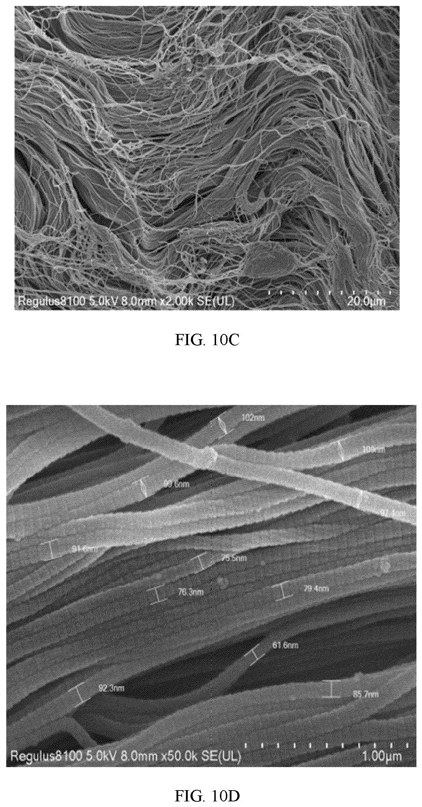

(7) The treated biological artificial blood vessels in step (5) were dehydrated, sputter coated with gold and imaged using scanning electron microscopy (SEM). FIG. 8, FIG. 9 and FIG. 10 showed SEM cross-sectional images, tunica intima images and tunica adventitia images of the biological artificial blood vessel at different magnifications, respectively. The average diameters were 74.04±11.37 nm for the cross-sectional fiber (n=10, FIG. 8c), 41.02±5.36 nm for the intimal fiber (n=10, FIG. 9c), and 84.89±12.96 nm for the adventitial fiber (n=10, FIG. 10d). A comparison of the diameters showed a significant difference between the cross-sectional fiber and the intimal fiber (P<0.01), a significant difference between the intimal fiber and the adventitial fiber (P<0.01), and no significant difference between the cross-sectional fiber and the adventitial fiber (P>0.05).

### Example 2

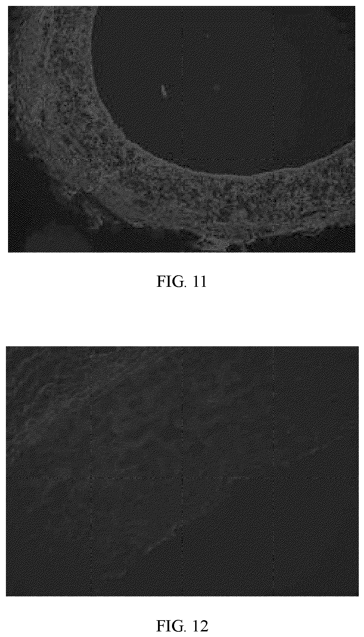

(1) The blood vessels obtained before and after the decellularization in Example 1 were sectioned and then subjected to DAPI staining. The results are shown in FIG. 11 and FIG. 12, where the blue fluorescence represents cell nuclei. The stained image of the vascular section before the decellularization showed a lot of cell nuclei, and the stained image of the vascular section after the decellularization showed no cell nuclei, indicating that the vessel wall cells were completely removed.

The biological artificial blood vessels before and after the decellularization were stained by 1) preparing 4 µm tissue sections, 2) air-drying at room temperature for 15 min, 3) fixing with fixative for 10 min and washing with PBS for 5 min, 4) staining with DAPI for 5 min, 5) washing with PBS for 10 min, and 6) sealing with an anti-fluorescence quenching sealing agent.

Unless otherwise stated, the following enzyme-treated blood vessels were obtained through decellularization using process A before the enzyme treatment.

(3) Suture Tension: The suture tension of the fresh bovine blood vessel in Example 1 was 206±7 g (n=8) and the suture tension of the elastin-free vascular graft in Example 1 (i.e., the blood vessels treated with ficin and DNase I in Step (3) and cross-linked with dialdehyde starch) was 201±11 g (n=8), showing no significant difference (P>0.05).

### Example 3

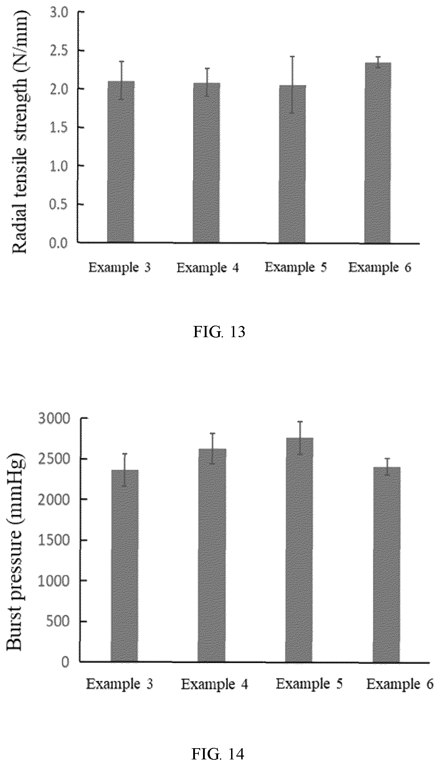

(1) Bovine superior rectal arteries were harvested from an 800 kg healthy cattle with inspection passed in a slaughterhouse. The diameter of the blood vessels was 4-8 mm. The fat and connective tissue surrounding the blood vessels were carefully removed using medical scissors without damaging the outer membrane of the blood vessels.
(2) Decellularization Treatment: The pretreated blood vessels were placed in a decellularizing agent of 3-[(3-cholamidopropyl)-dimethylammonium]-1-propanesulfonate inner salt (10 mmol/L), sodium dodecyl sulfate (2.5 mmol/L), NaCl (0.12 mmol/L) and NaOH (1 mol/L) in sterile deionized water, and treated at 20°C for 30 h. The solution was changed every 8 h for a total of 3 changes. After the treatment was completed, the blood vessels were transferred to 0.9% (V/V) physiological saline and washed at 37°C for 40 min to obtain decellularized blood vessels.
(3) Enzyme Treatment: The decellularized blood vessels in the previous step were placed in 0.9% (V/V) physiological saline containing Benzonase nuclease (15 KU/L), and treated at 37°C for 48 h. The solution was changed every 8 h for a total of 5 changes. After the treatment was completed, the blood vessels were transferred to 0.9% (V/V) physiological saline and washed at 37°C for 40 min. The Benzonase nuclease-treated blood vessels were treated with ficin (3 KU/L) in 0.9% (V/V) physiological saline at 37°C for 24 h. The solution was changed every 8 h for a total of 3 changes. After the treatment was completed, the blood vessels were transferred to 0.9% (V/V) physiological saline and washed for 40 min at 37°C to obtain enzyme-treated blood vessels.
(4) Cross-linking Treatment: Finally, the enzyme-treated blood vessels were placed in a solution containing 6% (wt) dialdehyde starch, treated at 25°C for 24 h, then transferred to 0.9% (V/V) physiological saline and washed for 40 min to obtain biological artificial blood vessels.
(5) Covalent Binding of Heparin: 800 mg of EDC and 400 mg of Sulfo-NHS were dissolved in 20 ml of MES buffer, 800 mg of heparin was dissolved in another 20 ml of MES buffer, the two solutions were mixed in an equal volume, and the resulting mixture was adjusted to a pH of 7 to obtain a heparin grafting agent. The biological artificial blood vessels obtained in the previous step were placed in the heparin grafting agent and shaken in the dark for 3 h to obtain biological artificial blood vessels coated with heparin. The heart bypass surgery was carried out.
(6) The treated blood vessels obtained in step (5) were subjected to radial tensile property and burst pressure tests according to the method in Example 2, and the test results showed that the radial tensile strength was 2.11±0.25 N/mm (n=8) (as shown in FIG. 13) and the burst pressure was 2364±203 mmHg (n=8) (as shown in FIG. 14). The DNA content was 26.42±2.21 ng/mg (n=8) as measured according to the method of Quantification of Residual DNA in Animal-Derived Biological Materials.
(7) The collagen and elastin of the biological artificial blood vessels obtained in step (5) were quantified, and the results showed that the collagen content was 80.32±9.06% of the dry weight of the blood vessel (n=8), and the elastin content was 0% of the dry weight of the blood vessel (n=8).

### Example 4

(1) Bovine median sacral arteries were harvested from a 900 kg healthy cattle with inspection passed in a slaughterhouse. The diameter of the blood vessels was 4-8 mm. The fat and connective tissue surrounding the blood vessels were carefully removed using medical scissors without damaging the outer membrane of the blood vessels.
(2) Decellularization Treatment: The pretreated blood vessels were placed in a decellularizing agent of 3-[(3-cholamidopropyl)-diethylammonium]-propanesulfonic acid (6 mmol/L), EDTA (8.0 mmol/L), NaCl (0.1 mmol/L) and NaOH (0.8 mol/L) in sterile deionized water, and were treated at 20°C for 30 h. The solution was changed every 8 h for a total of 3 changes. After the treatment was completed, the blood vessels were transferred to 0.9% (V/V) physiological saline and washed at 37°C for 40 min to obtain decellularized blood vessels.
(3) Enzyme Treatment: The decellularized blood vessels of the previous step were placed in 0.9% (V/V) physiological saline containing ficin (1000 KU/L), and treated at 37°C for 48 h. The solution was changed every 8 h for a total of 5 changes. After the treatment was completed, the blood vessels were transferred to 0.9% (V/V) physiological saline and washed at 37°C for 40 min to obtain enzyme-treated blood vessels.
(4) Cross-linking Treatment: Finally, the enzyme-treated blood vessels were placed in a solution containing 5% (wt) dialdehyde starch, treated at 25°C for 24 h, then transferred to 0.9% (V/V) physiological saline and washed for 40 min to obtain biological artificial blood vessels.
(5) Covalent Binding of Heparin: 1500 mg of EDC and 800 mg of Sulfo-NHS were dissolved in 20 ml of MES buffer, 1000 mg of heparin was dissolved in another 20 ml of MES buffer, the two solutions were mixed in an equal volume, and the resulting mixture was adjusted to a pH of 7 to obtain a heparin grafting agent. The biological artificial blood vessels obtained in the previous step were placed in the heparin grafting agent and shaken in the dark for 3 h to obtain biological artificial blood vessels coated with heparin.
(6) The treated blood vessels obtained in step (5) was subjected to radial tensile property and burst pressure tests according to the method in Example 2, and the test results showed that the radial tensile strength was 2.09±0.18 N/mm (n=8) (as shown in FIG. 13) and the burst pressure was 2627±185 mmHg (n=8) (as shown in FIG. 14). The DNA content was 42.57±2 ng/mg (n=8) as measured according to the method of Quantification of Residual DNA in Animal-Derived Biological Materials.
(7) The collagen and elastin of the biological artificial blood vessels obtained in step (5) were measured, and the results showed that the collagen content was 82.30±4.65% of the dry weight of the blood vessel (n=8), and the elastin content was 0% of the dry weight of the blood vessel (n=8).

### Example 5

(1) Porcine femoral veins were harvested from a 100 kg healthy pig with inspection passed in a slaughterhouse. The diameter of the blood vessels was 4-8 mm. The fat and connective tissue surrounding the blood vessels were carefully removed using medical scissors without damaging the outer membrane of the blood vessels.
(2) Decellularization Treatment: The pretreated blood vessels were placed in a decellularizing agent of 3-[(3-cholamidopropyl)-dimethylammonium]-1-propanesulfonate inner salt (8 mmol/L), EDTA (25 mmol/L), NaCl (0.12 mmol/L) and NaOH (1 mol/L) in sterile deionized water, and were treated at 25°C for 30 h. The solution was changed every 8 h for a total of 3 changes. After the treatment was completed, the blood vessels were transferred to 0.9% (V/V) physiological saline and washed at 37°C for 40 min to obtain decellularized blood vessels.
(3) Enzyme Treatment: The decellularized blood vessels in the previous step were placed in 0.9% (V/V) physiological saline containing DNase I (20 KU/L), and treated at 37°C for 48 h. The solution was changed every 8 h for a total of 5 changes. After the treatment was completed, the blood vessels were transferred to 0.9% (V/V) physiological saline and washed at 37°C for 40 min. The blood vessels were then placed in 0.9% (V/V) physiological saline containing ficin (100 KU/L) and treated at 37°C for 24 h. The solution was changed every 8 h for a total of 2 changes. After the treatment was completed, the blood vessels were transferred to 0.9% (V/V) physiological saline and washed at 37°C for 40 min to obtain enzyme-treated blood vessels.
(4) Cross-linking Treatment: Finally, the ficin-treated blood vessels were placed in a solution containing 3% (wt) dialdehyde starch and treated at 25°C for 24 h. Then they were transferred to 0.9% (V/V) physiological saline and washed for 40 min to obtain biological artificial blood vessels.
(5) Covalent Binding of Heparin: 2000 mg of EDC and 1000 mg of Sulfo-NHS were dissolved in 20 ml of MES buffer, 1500 mg of heparin was dissolved in another 20 ml of MES buffer, the two solutions were mixed in an equal volume, and the resulting mixture was adjusted to a pH of 7 to obtain a heparin grafting agent. The biological artificial blood vessels obtained in the previous step were placed in the heparin grafting agent and shaken in the dark for 3 h to obtain biological artificial blood vessels coated with heparin.
(6) The treated blood vessels obtained in step (5) were subjected to radial tensile property and burst pressure tests, and the test results showed that the radial tensile strength was 2.06±0.37 N/mm (n=8) (as shown in FIG. 13) and the burst pressure was 2764±201 mmHg (n=8) (as shown in FIG. 14). The DNA content was 39.31±1.8 ng/mg (n=8) as measured according to the method of Quantification of Residual DNA in Animal-Derived Biological Materials.
(7) The collagen and elastin of the vessel wall of the biological artificial blood vessels obtained in step (5) were quantified, and the results showed that the collagen content was 72.31±10.03% of the dry weight of the blood vessel (n=8), and the elastin content was 0% of the dry weight of the blood vessel (n=8).

### Example 6

(1) Sheep inferior mesenteric arteries were harvested from a healthy sheep with inspection passed. The diameter of the blood vessels was 4-8 mm. The fat and connective tissue surrounding the blood vessels were carefully removed using medical scissors without damaging the outer membrane of the blood vessels.
(2) Decellularization Treatment: The pretreated blood vessels were placed in a decellularizing agent of 3-[(3-cholamidopropyl)-dimethylammonium]-1-propanesulfonate inner salt (10 mmol/L), EDTA (30 mmol/L), NaCl (0.15 mmol/L) and NaOH (1.2 mol/L) in sterile deionized water, and were treated at 25°C for 30 h. The solution was changed every 8 h for a total of 3 changes. After the treatment was completed, the blood vessels were transferred to 0.9% (V/V) physiological saline and washed at 37°C for 40 min to obtain decellularized blood vessels.
(3) Enzyme Treatment: The decellularized blood vessels in the previous step were placed in 0.9% (V/V) physiological saline containing DNase I (50 KU/L), and treated at 37°C for 48 h. The solution was changed every 8 h for a total of 5 changes. After the treatment was completed, the blood vessels were transferred to 0.9% (V/V) physiological saline and washed at 37°C for 40 min to obtain enzyme-treated blood vessels.
(4) Cross-linking Treatment: Finally, the enzyme-treated blood vessels were placed in a solution containing 6% (wt) dialdehyde starch and treated at 25°C for 24 h. Then they were transferred to 0.9% (V/V) physiological saline and washed for 40 min to obtain biological artificial blood vessels.
(5) Covalent Binding of Heparin: 1600 mg of EDC and 800 mg of Sulfo-NHS were dissolved in 20 ml of MES buffer, 1600 mg of heparin was dissolved in another 20 ml of MES buffer, the two solutions were mixed in an equal volume, and the resulting mixture was adjusted to a pH of 7 to obtain a heparin grafting agent. The biological artificial blood vessels obtained in the previous step were placed in the heparin grafting agent and shaken in the dark for 3 h to obtain biological artificial blood vessels coated with heparin.
(6) The treated blood vessels obtained in step (5) were subjected to radial tensile property and burst pressure tests according to the methods in Example 2, and the test results showed that the radial tensile strength was 2.36±0.07 N/mm (n=8) (as shown in FIG. 13) and the burst pressure was 2412±105 mmHg (n=8) (as shown in FIG. 14). The DNA content was 48.13±1.47 ng/mg (n=8) as measured according to the method of Quantification of Residual DNA in Animal-Derived Biological Materials.
(7) The collagen and elastin of the vessel wall of the biological artificial blood vessels obtained in step (5) were quantified, and the results showed that the collagen content was 76.19±9.32% of the dry weight of the blood vessel (n=8), and the elastin content was 0% of the dry weight of the blood vessel (n=8).

### Example 7

Commercial artificial blood vessels (control group) and the biological artificial blood vessels coated with heparin prepared in Example 1 (experimental group) were implanted into the corresponding parts of experimental pigs, and the arteriovenous fistula model and common carotid artery replacement model were established using artificial blood vessels. Male experimental pigs, weighing 80-100 kg, received aspirin (100 mg/day) and Plavix (75 mg/day) for 5 days before surgery, and were fasted for 12-24 h before surgical anesthesia.

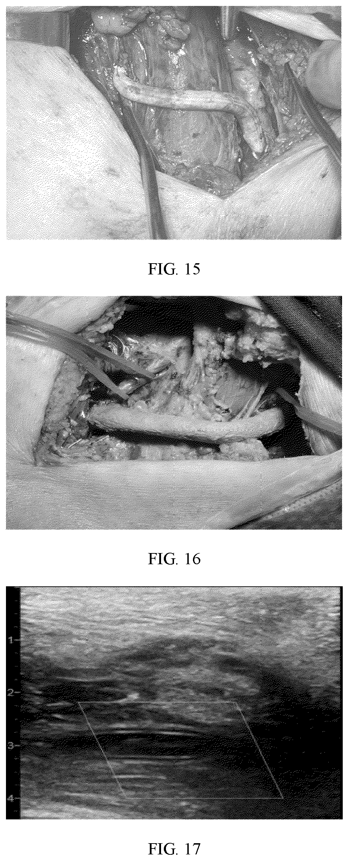

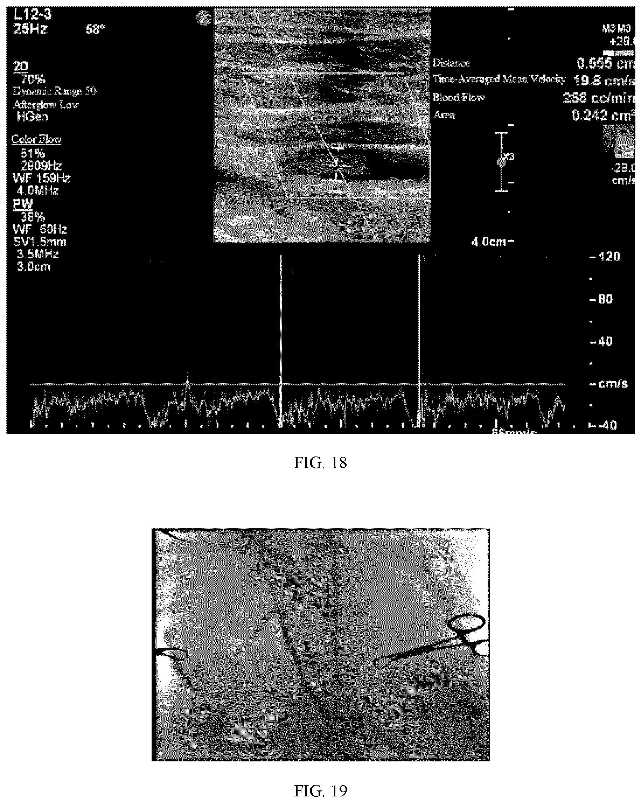

During the surgery, the animals were under general anesthesia, and the respiration, heart rate, and oxygen saturation of the animals were monitored in real time. The median skin of the neck was shaven, disinfected and applied with sterile drapes (three layers). The skin was incised in the middle and stopped from bleeding layer by layer. The right common carotid artery and right internal jugular vein were isolated, and the vessel diameter, flow rate and vessel wall thickness of the artery and vein were measured using an ultrasound probe during the surgery. Heparin was administered intravenously at a dose of 200 U/kg. Three minutes later, the artery was clamped with two Bulldog clamps, and a longitudinal incision of 8 mm was created on the right common carotid artery. The lumen of the biological artificial blood vessel was flushed with heparin-papaverine solution. The biological artificial blood vessel was anastomosed continuously using 6-0 Prolene suture, after which the biological artificial blood vessel was clamped and the anastomosis was checked for blood leakage. The internal jugular vein was clamped with two Bulldog clamps. The lumen of the biological artificial blood vessel was flushed with a heparin-papaverine solution, and the biological artificial blood vessel was anastomosed continuously using 6-0 Prolene suture. At the last stitch, the Bulldog clamps on the biological artificial blood vessel were removed to exhaust air, then the suture was tied, and the anastomosis was checked for blood leakage, as shown in FIG. 15 (control group) and FIG. 16 (experimental group). FIG. 17 and FIG. 18 show the flow rates at the anastomosis of the biological artificial blood vessel with both the artery and the vein as detected by ultrasound after the surgery. The surgical field was carefully inspected, and the incision was sutured layer by layer. As shown in FIG. 19, the patency of the vascular graft of the arteriovenous fistula pig model was inspected by postoperative angiography. After recovered from anesthesia, the animals were returned to the animal facility for observation, and subsequent care and management were carried out according to standard operating procedures for animal experiments. Ceftriaxone sodium (2.0 g) was administered twice daily for one week after the surgery. Aspirin (100 mg) was administered once daily, and Plavix (75 mg) was also administered once daily till the end of the three-month observation period

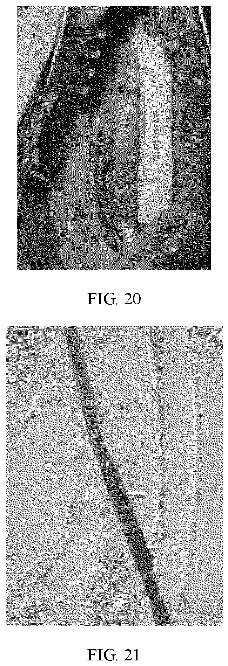

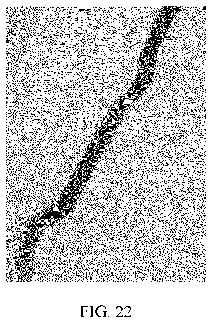

The biological artificial blood vessel coated with heparin used in the right common carotid artery replacement pig model is shown in FIG. 20. FIG. 21 shows the angiography immediately performed after the surgery, indicating that the blood vessel was patent. The follow-up time points for postoperative ultrasound detection and digital subtraction angiography (DSA) are shown in Table 3. The angiographic follow-up showed that the biological artificial blood vessel and the autologous vessel near the anastomosis were patent, as shown in FIG. 22.

### 1) Postoperative follow-up

**Table 3 Follow-up time points for ultrasound detection and DSA of artificial vessel arteriovenous fistula model and common carotid artery replacement model**

| Follow-up time point for ultrasound detection | Follow-up time point for DSA |
|---|---|
| Before surgery | Before surgery |
| Immediately after surgery | Immediately after surgery |
| 1 month after surgery | 1 month after surgery |
| 2 months after surgery | 2 months after surgery |
| 3 months after surgery | 3 months after surgery |
| 6 months after surgery | 6 months after surgery |

2) Postoperative complications and accidents: hematoma, swelling, redness and abscess of the neck and pulmonary embolism were observed after the surgery.

3) Animal Sacrifice and Sampling: After the observation period ended, the experimental animals were sacrificed in accordance with the standard operating procedures for experimental animals. A comprehensive examination was conducted, including an assessment of the overall condition of the animals and major organs (heart, liver, spleen, lung and kidney). A median incision was made on the neck skin. The right common carotid artery and right internal jugular vein were isolated, and administered with heparin at a dose of 200 U/kg. Three minutes later, the right common carotid artery and right internal jugular vein were each clamped with two Bulldog clamps. "I-shaped" samples of the artificial vessels, retaining 2 cm of blood vessel near the arterial anastomosis and 5 cm of blood vessel near the venous anastomosis respectively, were harvested, washed with physiological saline, and fixed with 4% paraformaldehyde. After the necropsy and sample collection of the experimental animals were completed, the animal carcasses were preliminarily processed according to standard operating procedures and transported to an animal carcass disposal facility for harmless incineration.

4) Experimental Evaluation: The vessel patency and the degree of stenosis wre evaluated using ultrasound and DSA. The blood vessels were sectioned and stained. The percentage of the neointimal area in relation to the luminal area was calculated for the arterial anastomosis, the midsection of the artificial vessel, the venous anastomosis, and both the proximal and distal vein segments.

The above is only the preferred embodiments of the present disclosure. It should be noted that a person skilled in the art can make several changes and modifications without departing from the principle of the present disclosure, and these changes and modifications should also be regarded as the protection scope of the present disclosure.

## Claims

1. A method for producing a biological artificial blood vessel having a nanostructure and low immunogenicity, comprising:
S1) decellularizing a pretreated animal blood vessel to obtain a decellularized blood vessel,
S2) treating the decellularized blood vessel with an enzyme solution to obtain an enzyme-treated blood vessel, wherein the enzyme solution comprises a nuclease and/or a biological enzyme, and
S3) crosslinking the enzyme-treated blood vessel with a cross-linking agent to obtain the biological artificial blood vessel having a nanostructure and low immunogenicity.

2. The method according to claim 1, wherein the animal blood vessel is an artery or a vein of a large animal, wherein the large animal is selected from the group consisting of a pig, a sheep, a dog, a cow and a horse;
the animal blood vessel is selected from the group consisting of an aorta, a pulmonary artery, a superior artery, an inferior artery, a common carotid artery, an internal jugular vein, an external jugular vein, a femoral artery, a femoral vein, an iliac artery, an iliac vein, a superior mesenteric artery, an inferior mesenteric artery, a rectal artery, a median sacral artery and a lower limb artery;
the decellularization in step S1) is carried out using a decellularizing agent, the decellularizing agent comprises a detergent or a combination of a detergent and a chelating agent, wherein the detergent in the decellularizing agent has a concentration of 0.01 to 500 mmol/L, and the chelating agent in the decellularizing agent has a concentration of 0.01 to 500 mmol/L; and
the decellularization is carried out at a temperature of 10°C to 38°C for 2 to 72 h, and the decellularizing agent is changed every 1 to 24 h during the decellularization.

3. The method according to claim 2, wherein in the case that the decellularizing agent comprises a detergent, the detergent is one or more selected from the group consisting of a non-ionic detergent, an anionic detergent and a cationic detergent, and an amphoteric detergent;
in the case that the decellularizing agent comprises a combination of a detergent and a chelating agent, the detergent is one or more selected from the group consisting of a non-ionic detergent, an anionic detergent, a cationic detergent and an amphoteric detergent,
wherein, the non-ionic detergent is one or more selected from the group consisting of polyethylene glycol, polyethylene glycol octylphenyl ether, polyol, polyoxyethylene fatty alcohol ether and polyoxyethylene alkyl phenol ether,
the anionic detergent is one or more selected from the group consisting of sodium dodecyl sulfate, lithium dodecyl sulfate, sodium dodecyl sulfonate, sodium cholate and sodium deoxycholate,
the cationic detergent is selected from the group consisting of benzalkonium bromide, cetyltrimethylammonium bromide and a combination thereof,
the amphoteric detergent is selected from the group consisting of 3-[(3-cholamidopropyl)-dimethylammonium]-1-propanesulfonate inner salt, n-tetradecyl-N,N-dimethyl-3-ammonium-1-propanesulfonate and a combination thereof, and
the chelating agent is selected from the group consisting of an inorganic chelating agent, an organic chelating agent and a combination thereof,
wherein, the inorganic chelating agent is one or more selected from the group consisting of sodium tripolyphosphate, sodium hexametaphosphate and sodium pyrophosphate,
the organic chelating agent is one or more selected from the group consisting of amino triacetic acid, ethylenediaminetetraacetic acid, ethylene glycol bis(tetraacetic acid), ethylenediamine diacetic acid, cyclohexane diaminetetraacetic acid, S,S-ethylenediamine disuccinic acid, diethyl triacetic acid, diethylenetriamine pentaacetic acid and a salt thereof, citric acid, tartaric acid, gluconic acid, hydroxyethyl ethylenediamine triacetic acid and dihydroxyethyl glycine; and
the decellularizing agent further comprises sodium chloride, sodium hydroxide and water,
wherein, the sodium chloride in the decellularizing agent has a concentration of 0.01 to 1.0 mmol/L, and
the sodium hydroxide in the decellularizing agent has a concentration of 0.1 to 2.0 mmol/L.

4. The method according to claim 1, wherein the nuclease in the enzyme solution has a concentration of 1 to 50,000 KU/L, and/or the biological enzyme in the enzyme solution has a concentration of 1 to 50,000 KU/L,
the enzyme solution further comprises 1 to 50 wt% human serum or animal serum,
the enzyme solution further comprises physiological saline or buffer, and
the enzyme treatment in step S2) is carried out at a temperature of 36°C to 37°C for 2 to 72 h, and the enzyme solution is changed every 1 to 24 h during the enzyme treatment.

5. The method according to claim 1, wherein after the enzyme treatment, step S2) further comprises treating the blood vessel with a solution comprising a biological enzyme to obtain an enzyme-treated blood vessel,
the nuclease in the enzyme treatment is selected from the group consisting of DNase, RNase and a combination thereof, the biological enzyme in both the enzyme treatment and the solution comprising a biological enzyme is one or more selected from the group consisting of pepsin, lipase, trypsin, cathepsin, papain, ficain and subtilisin, the biological enzyme in the solution comprising a biological enzyme has a concentration of 1 to 50,000 KU/L, and the solution comprising a biological enzyme further comprises a solvent selected from the group consisting of physiological saline, buffer and a special solution for enzyme preparation,
the treatment with the solution comprising a biological enzyme is carried out at a temperature of 36°C to 37°C for 0.25 to 48 h, and the solution comprising a biological enzyme is changed every 1 to 24 h during the treatment.

6. The method according to claim 1, wherein the cross-linking agent is one or more selected from the group consisting of a glutaraldehyde solution, an oxidized starch solution, a dialdehyde starch solution and a Jeffamine buffer,
the glutaraldehyde solution has a concentration of 0.1 to 30 wt%,
the oxidized starch solution has a concentration of 0.01 to 10 wt%,
the dialdehyde starch solution has a concentration of 0.01 to 10 wt%, and
the Jeffamine buffer has a concentration of 0.01 to 1.0 mol/L.

7. The method according to claim 1, wherein after the crosslinking, step S3) further comprises performing covalent binding of heparin to the cross-linked blood vessel by a chemical method to obtain the biological artificial blood vessel.

8. The method according to claim 7, wherein the covalent binding of heparin to the cross-linked blood vessel by a chemical method is carried out using a solution of carbodiimide, N-hydroxysulfosuccinimide and heparin in MES buffer, and the heparin in the solution for the covalent binding of heparin has a concentration of 1 to 200 mg/ml.

9. A biological artificial blood vessel having a nanostructure and low immunogenicity produced by the method according to any one of claims 1 to 8, comprising a vascular collagen or a nanofibrous structure formed by vascular collagen and elastin.

10. The biological artificial blood vessel having a nanostructure and low immunogenicity according to claim 9, wherein the vascular collagen includes type I collagen, type III collagen and/or type IV collagen,
in the case that the biological artificial blood vessel comprises only the vascular collagen, the vascular collagen has a content of 30% to 85% of the dry weight of the biological artificial blood vessel, and
in the case that the biological artificial blood vessel comprises the vascular collagen and elastin, the vascular collagen has a content of 20% to 55% of the dry weight of the biological artificial blood vessel, and the elastin has a content of 20% to 55% of the dry weight of the biological artificial blood vessel.

11. Use of the biological artificial blood vessel having a nanostructure and low immunogenicity produced by the method according to any one of claims 1 to 8 or the biological artificial blood vessel having a nanostructure and low immunogenicity according to claim 9 or 10 in the manufacture of a vascular graft material for hemodialysis vascular access in chronic renal failure, a vascular graft material for arterial trauma of lower limbs, a vascular graft material for peripheral artery bypass grafting or a vascular graft material for coronary artery bypass grafting.

12. Use of the biological artificial blood vessel having a nanostructure and low immunogenicity produced by the method according to any one of claims 1 to 8 or the biological artificial blood vessel having a nanostructure and low immunogenicity according to claim 9 or 10 in the manufacture of a vascular graft material for vascular access of pateints with chronic haemodialysis; wherein, the biological artificial blood vessel is used as a replacement repair graft material for failure, infection or aneurys formation after chronic dialysis autologous arteriovenous fistula creation or artificial vascular fistula creation.
